# FASCICULE DE BREVET EUROPEEN

(11) **EP 3 094 612 B1**
(45) Date de publication et mention de la délivrance du brevet: **04.08.2021**
(21) Numéro de dépôt: 15704043.7
(22) Date de dépôt: 13.01.2015
(51) Int. Cl.: C07C 17/20, C07C 21/18, C07C 17/383

(54) **PROCEDE DE PRODUCTION DU E-1-CHLORO-3,3,3-TRIFLUOROPROPENE A PARTIR DU 1,1,3,3-TETRACHLOROPROPENE**
VERFAHREN ZUR HERSTELLUNG VON E-1-CHLORO-3,3,3-TRIFLUOROPROPEN AUS 1,1,3,3-TETRACHLOROPROPEN
METHOD FOR PRODUCING E-1-CHLORO-3,3,3-TRIFLUOROPROPENE FROM 1,1,3,3-TETRACHLOROPROPENE

(30) Priorité: 13.01.2014 US 201414153500; 17.01.2014 FR 1450381
(43) Date de publication de la demande: 23.11.2016
(73) Titulaire: Arkema France, 92700 Colombes (FR)
(72) Inventeur: PIGAMO, Anne, 69340 Francheville (FR); COLLIER, Bertrand, 69230 Saint-Genis-Laval (FR); BONNET, Philippe, 69007 Lyon (FR); WISMER, John, Washington Crossing, Pennsylvania 18977 (US)
(74) Mandataire: Arkema Patent
(86) Numéro de dépôt international: PCT/FR2015/050072
(87) Numéro de publication internationale: WO 2015/104517

(56) Documents cités:
- EP-A1- 0 940 382
- WO-A1-2010/059496
- WO-A1-2010/111067
- US-A- 5 684 219
- US-A- 5 877 359
- US-A1- 2011 201 853
- US-A1- 2013 261 354

## Description

### DOMAINE DE L'INVENTION

La présente invention concerne un procédé de fabrication continue de E-1-chloro-3,3,3-trifluoropropène (E-1233zd) comprenant au moins une étape de fluoration en phase liquide du 1,1,3,3-tetrachloropropène (1230za).

### ARRIERE-PLAN TECHNIQUE

Le E-1-chloro-3,3,3-trifluoropropène (E-1233zd) peut être fabriqué par fluoration du 1,1,1,3,3-pentachloropropane (240fa). Par exemple, les documents US 8,436,217 et US 8,426,656 décrivent la fluoration du 240fa en E-1233zd en phase liquide, en présence de catalyseurs adaptés tels que TiCl₄ ou une combinaison de TiCl₄ et SbCl₅.

Le document FR2768727 enseigne également que la fluoration du 240fa ou du 1230za est réalisable en présence d'un catalyseur tel que TiCl₄.

Les documents US2010/0191025 et US 6,166,274 décrivent également l'utilisation d'un catalyseur pour la fluoration en phase liquide du 1230za afin d'obtenir le E-1233zd : un catalyseur à base de liquide ionique pour le premier document et un acide triflique ou trifluoroacétique pour le deuxième document.

Le document US 2012/0059199 décrit la fluoration en phase liquide de 240fa en l'absence de catalyseur. Ce document enseigne qu'un désavantage de procédé en phase liquide non catalysé est le faible taux de conversion de la réaction. Plusieurs réacteurs en série sont donc nécessaires pour augmenter le taux de conversion global, chacun des réacteurs apportant sa contribution à l'avancement du taux de conversion.

Le document US 2013/0211154 décrit l'utilisation d'une pression de réaction élevée associée à un réacteur de fluoration agité pour pouvoir augmenter le taux de conversion dans un procédé en phase liquide non catalysé du 240fa. Toutefois, il n'y a aucune précision du taux de conversion.

Le document US 6,987,206 décrit la possibilité d'obtenir le E-1233zd à partir du 1230za comme intermédiaire sans indication de conditions opératoires.

La réaction non catalysée en phase liquide de fluoration du 1230za est exemplifiée dans le document US 5,616,819. La pression utilisée est de 200 psi (14 bar) et engendre la formation d'oligomères malgré la courte durée de l'essai batch.

Le document US 5,877,359 présente la fluoration non catalysée en phase liquide du 1230za. Les exemples montrent qu'un ratio molaire très élevé de 166 a été utilisé pour obtenir une conversion complète sur un essai batch de courte durée. Lorsque le ratio molaire est diminué à 12,6, une pression de 600psig (42 bar) est appliquée. D'autre part, les conditions opératoires d'un procédé continu extrapolable ne sont donc pas définies : ratio molaire HF/1230za, température de reflux, nature des sous-produits à distiller. La productivité ou la stabilité dans le temps d'un procédé basé sur cette réaction ne sont pas décrites non plus.

Il existe toujours un besoin de mettre au point un nouveau procédé de production continu et extrapolable de E-1233zd en évitant des conditions opératoires contraignantes telles qu'une pression excessive, un ratio molaire élevé ou une agitation dans le réacteur de fluoration qui pourraient conduire à une perte de sélectivité par formation d'oligomères ou de produits surfluorés.

### RESUME DE L'INVENTION

La présente invention permet de surmonter les inconvénients de l'état de la technique. Elle fournit plus particulièrement un nouveau procédé de production de E-1-chloro-3,3,3-trifluoropropène.

Ceci est accompli grâce à la mise en œuvre simplifiée de la fluoration du 1230za en E-1233zd par le fluorure d'hydrogène en phase liquide en l'absence de catalyseur.

### DESCRIPTION DE MODES DE REALISATION DE L'INVENTION

Selon la présente invention, le procédé comprend au moins une étape au cours de laquelle le 1,1,3,3-tetrachloropropène réagit avec de l'acide fluorhydrique anhydre en phase liquide, en l'absence de catalyseur, avec un ratio molaire HF/1,1,3,3-tetrachloropropène compris entre 9 et 20, à une température comprise entre 50 et 150°C et une pression absolue comprise entre 1 et 20 bar, caractérisé en ce que la réaction de fluoration est mise en oeuvre dans un réacteur non agité.

Le ratio molaire HF/1,1,3,3-tetrachloropropène est de préférence compris entre 9 et 14 et préférentiellement entre 9 et 12. Le ratio molaire HF/1,1,3,3-tetrachloropropène inclus la partie d'HF recyclée et est de préférence mesuré à l'entrée du réacteur. La présence d'un excès d'HF par rapport au composé organique permet d'évacuer rapidement le E-1233zd formé par entrainement azéotropique et permet d'éviter ainsi la formation consécutive de produits surfluorés, comme en particulier le 1,3,3,3-tétrafluoropropène, ou 1234ze, et le 1,1,1,3,3-pentafluoropropane, ou 245fa. La pureté du produit final est ainsi améliorée grâce à la diminution de la quantité d'impuretés formées dès l'étape réactionnelle.

La température de réaction est de préférence comprise entre 80 et 120, et avantageusement comprise entre 90 et 110°C.

La réaction de fluoration est de préférence mise en œuvre à une pression comprise entre 5 et 20 bars, préférentiellement entre 5 et 15 bars, encore plus préférentiellement entre 7 et 15 bars et avantageusement comprise entre 7 et 12 bars. La réaction de fluoration est mise en œuvre dans un réacteur non agité.

Le procédé selon la présente invention peut être mis en œuvre en continu, discontinu ou batch. On préfère opérer en continu.

Le procédé selon la présente invention offre l'avantage d'obtenir un rendement et une sélectivité très intéressants tout en utilisant des conditions douces. En outre, ces résultats peuvent être obtenus à l'aide d'un réacteur unique.

De préférence, lorsqu'il est fait référence à des intervalles, les expressions « compris entre...et... » excluent les bornes de l'intervalle.

### MODE DE REALISATION DE L'INVENTION

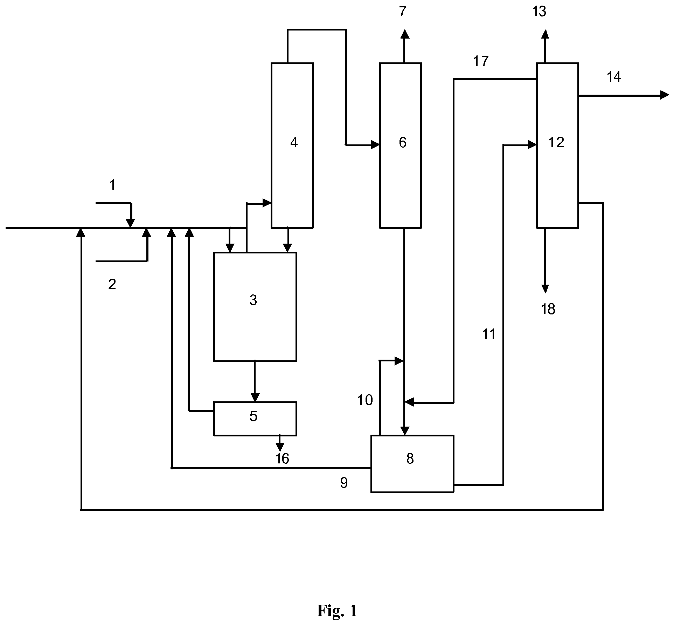

La **figure 1** représente de manière schématique un mode de réalisation du procédé selon l'invention et une installation pour sa mise en œuvre.

Sauf mention contraire, l'ensemble des pourcentages indiqués ci-dessous sont des pourcentages massiques.

L'invention prévoit la fluoration de 1230za en E-1233zd par du fluorure d'hydrogène en phase liquide, en l'absence de catalyseur.

En faisant référence à la figure 1, l'installation comprend un réacteur catalytique 3 pour la mise en œuvre de la réaction de fluoration du 1230za en E-1233zd.

Le réacteur 3 est alimenté par une conduite d'amenée de 1,1,3,3-tétrachloropropène 2 et une conduite d'amenée de fluorure d'hydrogène 1. Des moyens de chauffage sont de préférence prévus pour préchauffer les réactifs avant leur arrivée dans le réacteur 3.

Les conduites d'amenée précitées peuvent alimenter le réacteur 3 séparément ou bien peuvent être connectées ensemble en amont du réacteur pour alimenter celui-ci en mélange de réactifs.

Le réacteur 3 est de préférence un réacteur métallique. Le métal du réacteur pourra être de l'acier ou de l'acier inoxydable. Toutefois, d'autres matériaux comme un acier inoxydable superausténitique ou un alliage à base nickel passivable pourront être utilisés. L'absence de catalyseur pour la réaction est un avantage qui permet d'éviter des phénomènes de corrosion connus de l'homme de l'art lorsqu'un catalyseur de fluoration est utilisé dans ce type de réacteur.

L'ensemble des autres équipements de l'installation et notamment l'ensemble des colonnes de séparation ou colonnes de distillation peuvent être en métal.

Le réacteur 3 peut comprendre une enveloppe chauffante permettant de porter le mélange réactionnel à la température souhaitée entre 50 et 150°C, de préférence entre 80 et 120°C et de manière avantageuse entre 90 et 110°C.

Une conduite de prélèvement permet de purger une quantité de produits indésirables de haut poids moléculaire qui auraient pu se former au cours de la réaction de fluoration. Ce flux contient également de l'HF et des composés organiques valorisables qui sont séparés par un traitement spécifique 5 avant d'être retournés au réacteur. Ce traitement implique des technologies connues de l'homme de l'art comme la décantation ou la distillation azéotropique, et de préférence une combinaison des deux. Le flux 16 correspond aux composés lourds non valorisables qui doivent être éliminés du procédé.

Une conduite de prélèvement de produits issus de la réaction est connectée en sortie du réacteur 3. Cette conduite transporte un flux contenant le produit désiré (E-1233zd), du chlorure d'hydrogène, du fluorure d'hydrogène et des coproduits et sous-produits de la réaction.

La conduite de prélèvement de produits issus de la réaction alimente une unité de séparation préliminaire 4, qui est de préférence une colonne de distillation munie d'un système de reflux en tête. Cette unité de séparation préliminaire assure une première séparation du HF du reste des produits issus de la réaction.

En tête de l'unité de séparation préliminaire 4 est connectée une première conduite intermédiaire, qui est destinée à la collecte des produits issus de la réaction restants, et alimente une unité de séparation 6 destinée à la séparation du chlorure d'hydrogène, qui est un coproduit de la réaction. La composition issue de l'unité de séparation intermédiaire 4 comprend typiquement 43% E-1233zd, 15-18% HF, 35-40% HCl, le complément étant constitué de Z-1233zd, (Z+E)-1234ze, 243fc, 245fa et d'isomères 1232 et/ou 1231

Des moyens de refroidissement peuvent être prévus sur la première conduite intermédiaire de sorte que la première unité de séparation opère à la température souhaitée.

L'unité de séparation 6 est de préférence une colonne de distillation munie d'un rebouilleur en pied et d'un système de reflux en tête. Elle peut par exemple être opérée à une pression légèrement inférieure à celle du réacteur 3. En tête de la première unité de séparation est connectée une conduite de prélèvement de chlorure d'hydrogène par laquelle est prélevé un flux 7 comprenant majoritairement du chlorure d'hydrogène. Des traces de E-1233zd ou de coproduits légers comme le 245fa ou E-1234ze peuvent être présentes dans ce flux.

Le HCl produit est préférablement valorisé sous forme de HCl solution après absorption adiabatique ou isotherme dans l'eau. L'HCl peut être purifié par passage du gaz dans des tours d'alumine pour avoir une qualité d'analyse souhaitée.

En pied de l'unité de séparation 6 est connecté un système de séparation 8 qui va permettre la séparation du HF et des autres produits organiques. La composition issue de l'unité de séparation 6 comprend typiquement entre 25 et 30% de HF, 70% de E-1233zd, entre 1 et 5% de composés sous-produits de la réaction. Ce système de séparation pourra être constitué d'une première unité de séparation de phases constituée d'un décanteur, mis en œuvre à une température avantageusement comprise entre - 50 et 50°C, de préférence entre -20°C et 10°C. La phase riche en HF pourra être ainsi amenée vers une unité de séparation qui est de préférence une colonne de distillation azéotropique dont la fraction en pied de colonne est enrichie en HF avant d'être recyclée au réacteur 3 selon la conduite 9 (99,7% HF, 0.3% E-1233zd). La fraction azéotropique 10 collectée en tête (69% E-1233zd, 27% HF, le reste étant composé d'HCl et de traces d'autres organiques) est recyclée vers l'unité de séparation 8.

La phase riche en composés organiques sera collectée par la conduite 11 et pourra être traité, selon la Figure 1, par un train aval d'une unité de purification 12 comprenant au moins une colonne de distillation azéotropique supplémentaire permettant de finaliser la séparation du HF et une unité de purification finale permettant d'obtenir le E-1233zd avec une pureté supérieure ou égale à 98 % (flux 14 de la figure1). De préférence, avant l'étape de purification, la phase issue de l'unité de séparation 8 a une composition d'environ 90-95 % E-1233zd, 3-5 % HF, 1-5% Z-1233zd et 0,1-2% de coproduits et de produits intermédiaires.

Selon un mode de réalisation, le procédé comprend deux distillations azéotropiques prévues : une colonne azéotropique intégrée dans le système de séparation 8 après le décanteur et une colonne azéotropique dans l'unité de purification 12.

L'unité de purification 12 comprend de préférence une première colonne à distiller pour éliminer les produits légers (245fa, E-1234ze ou des traces résiduelles d'HCl) qui sont évacués du procédé par la conduite 13.

Le flux gazeux résultant de cette première distillation est ensuite traité par une colonne azéotropique pour finaliser la séparation de l'HF. Une composition azéotropique est ainsi collectée dans la conduite 17 et recyclée vers l'unité de séparation 8. La composition de ce flux est proche de celle du flux de la conduite 10, c'est-à-dire un mélange azéotropique de HF et de E-1233zd.

Le flux gazeux résultant est finalement traité par au moins une colonne de purification, de préférence deux colonnes, pour éliminer une fraction contenant majoritairement l'isomère cis-1233zd et des composés intermédiaires (par exemple les isomères 1231, 1232, 241, 242, 243...) qui sont recyclés au réacteur par la conduite 15 et une autre fraction contenant des composés non valorisables (lourds, 1223xd, ..) qui sont éliminés par la conduite 18.

Le produit final E-1233zd est ensuite collecté par la conduite 14 avec une pureté supérieure ou égale à 98 %, avantageusement supérieure ou égale à 99 %, et très avantageusement supérieure ou égale à 99,9 % en poids.

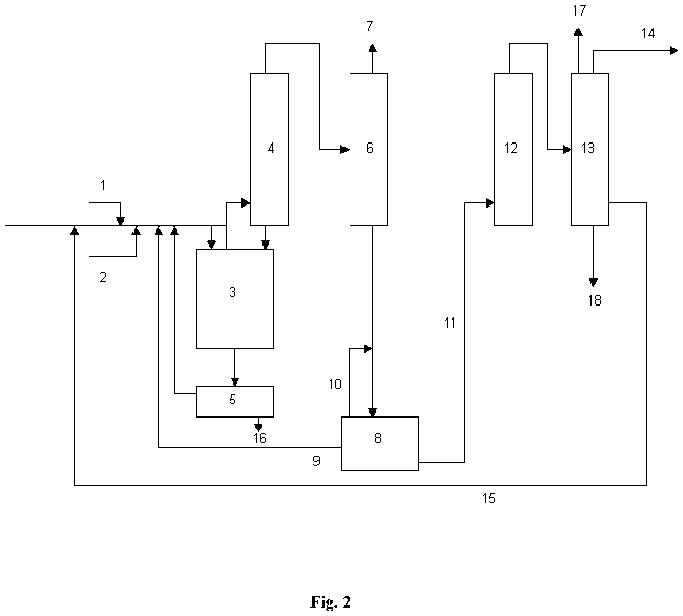

Selon un mode de réalisation préféré référencé dans la Figure 2, la phase riche en composés organiques sera collectée par la conduite 11 (environ 90-95 % E-1233zd, 3-5 % HF, 1-5% Z-1233zd et 0,1-2% de coproduits et produits intermédiaires) et pourra être traité par un système de lavage par absorption 12 puis une étape d'élimination finale 13 permettant de finaliser la séparation du HF et d'obtenir le E-1233zd avec une pureté supérieure ou égale à 98 % (Flux 14 de la Figure 2).

Le système de lavage 12 est constitué de préférence d'un premier lavage à l'eau qui permet d'absorber le HF résiduel. La température de ce lavage est maintenue au-dessus du point de rosée du mélange organique. Le système de lavage est complété ensuite par une deuxième colonne de lavage alimentée par une solution caustique (KOH, NaOH, ..) qui permet de finaliser la neutralisation du flux. Le flux organique est ensuite séché (à l'aide par exemple de tamis moléculaires) puis condensé avant d'être conduit vers le train final de purification.

A l'issue de ce lavage, la composition du flux ne contient plus de HF et la répartition des composés organiques est typiquement la suivante : 93-94% de E-1233zd, 3,5-4% d'isomère Z-1233zd, 1% de 1232zd, moins de 1% de 1234ze et moins de 1% de 245fa.

L'unité de purification 13 comprend de préférence une première colonne à distiller pour éliminer les produits légers (245fa, E-1234ze) qui sont évacués du procédé par la conduite 17.

Le flux gazeux résultant est finalement traité par au moins une colonne de purification, de préférence deux colonnes, pour éliminer une fraction contenant majoritairement l'isomère cis-1233zd et des composés intermédiaires (par exemple les isomères 1231, 1232, 241, 242, 243...) qui sont recyclés au réacteur par la conduite 15 et une autre fraction contenant des composés non valorisables (lourds, 1223xd, ..) qui sont éliminés par la conduite 18.

Le produit final E-1233zd est ensuite collecté par la conduite 14 avec une pureté supérieure ou égale à 98 %, avantageusement supérieure ou égale à 99 %, et très avantageusement supérieure ou égale à 99,9 % en poids.

### EXEMPLES

Les exemples suivants illustrent l'invention sans la limiter.

Une première étape consiste à préparer la matière première. Le 1,1,3,3-tetrachloropropène est obtenu par déshydrochloration du 1,1,1,3,3-pentachloropropane en présence de chlorure ferrique anhydre.

### Exemple 1 : Préparation du 1230za par déshydrochloration du 240fa

Dans un réacteur en verre équipé d'une double enveloppe et d'un reflux, on introduit 1441,6g de 1,1,1,3,3-pentachloropropane, de pureté de 99,6%. Le ciel du réacteur est balayé par un débit d'azote de 4l/h pour inerter l'atmosphère. On introduit ensuite 14,4g de chlorure ferrique anhydre avant de mettre en route l'agitation à 800 tours/min. Le reflux est alimenté par un fluide maintenu à 20°C. La sortie gaz du condenseur est reliée à un barboteur à eau qui permet de piéger l'HCl qui se dégage au cours de la réaction de déshydrochloration. Le mélange est ensuite chauffé entre 75 et 80°C pendant plusieurs heures (environ 4 heures) jusqu'à ce que le dégagement gazeux cesse. 1195.6g de solution résultante sont vidangés du ballon. Le mélange obtenu est filtré pour éliminer le chlorure ferrique en suspension puis analysé en chromatographie phase gazeuse.

**Tableau 1- déshydrochloration du 240fa : composition du mélange**

| Composé (mol%) | Avant réaction | Après réaction |
|---|---|---|
| 1230za | 0,055 | 92,613 |
| 250 | 0,035 | 0,025 |
| 240fa | 99,58 | 6,062 |
| C₂Cl₆ | 0,051 | 0,052 |
| 240db | 0,157 | 0,159 |

### Exemple 2 : distillation du 1230za

Le 1230za de faible pureté est ensuite soumis à une distillation classique de laboratoire impliquant une colonne de 10 plateaux, un réfrigérant, une pompe à vide, un ballon et des ballons de réception. La distillation est réalisée sous un vide de 25 mbar, le produit 1230za a alors un point d'ébullition de 53°C. On obtient une matière première de bonne pureté ayant la composition suivante: 99,33% de 1230za, 0,02% 250fa, 0,15% 240fa, 0,009% C₂Cl₆ et 0,001% 240db.

### Exemple 3 : fluoration continue en phase liquide du 1230za avec un ratio molaire de 9 à l'entrée du réacteur

L'équipement utilisé consiste en un autoclave d'une capacité de 1 litre avec une double enveloppe, fabriqué en acier inoxydable 316L. Il est pourvu de moyens de mesure de température et de pression. Des ouvertures au sommet de l'autoclave permettent l'introduction des réactifs et l'enlèvement des produits. Un condenseur est prévu au sommet, ainsi qu'une vanne pour la régulation de la pression. Le condenseur est contrôlé en température au moyen d'un bain thermostaté indépendant. Sa fonction est de renvoyer le dans le réacteur une partie du HF non réagi et des intermédiaires.

Les produits de la réaction sont extraits en continu lors de la réaction. Le flux de gaz de sortie passe dans un dispositif de lavage qui collecte les hydracides HF et HCl, puis est refroidi dans de l'azote liquide. La répartition molaire des produits du gaz de sortie est analysée périodiquement, par GPC (chromatographie en phase gazeuse).

A la fin de l'essai, le milieu réactionnel est dépressurisé et lentement chauffé afin d'évacuer le HF résiduel. Pendant cette période de dégazification, les composés organiques éventuellement entraînés sont également récupérés, après un passage dans le dispositif de lavage afin d'éliminer HF et HCl du flux gazeux. Dans une dernière étape, l'autoclave est ouvert et vidé.

La matière première préparée dans l'exemple 2 est utilisée pour une réaction de fluoration.

Une quantité de 300g d'HF est introduite dans l'autoclave. La température du réacteur est ajustée à 92-93°C dans la phase liquide. La régulation de la pression est effectuée à 10 bars abs. Les réactifs sont ensuite introduits avec les débits suivants : 20 g/h de 1230za et 20 g/h d'HF. Le rapport molaire d'HF sur le composé organique est donc de 9. L'établissement d'un équilibre massique correct entre l'entrée et la sortie est régulièrement vérifié. La composition du flux de sortie est suivie par analyse GPC, et reportée dans le tableau 2 :

**Tableau 2 - composition molaire du gaz de sortie (débit d'entrée de 1230za de 20g/h)**

| Temps | Composition molaire en sortie | | | |
|---|---|---|---|---|
| | F1233zd-E | F1233zd-Z | F1234ze(E+Z) | F245fa |
| 5,5 h | 90,6 % | 3,9 % | 1,4 % | 2,2% |
| 23 h | 92,1 % | 3,7 % | 1,5 % | 1,4 % |
| 29,2 h | 91,6 % | 3,7 % | 1,6 % | 1,4 % |
| 46,7 h | 92,4 % | 3,7 % | 1,5 % | 1,1% |
| 53,7 h | 92,1 % | 3,6 % | 1,6 % | 1,2 % |

La quantité d'impuretés surfluorées 1234ze (E+Z) et 245fa restent supérieures à 1% tout au long de l'essai.

Le complément de la composition est constitué de produits intermédiaires (1231, 1232,, 242, 243) et / ou de produits non identifiés.

La productivité du système réactionnel en F1233zd-E est de 0,31 mol/h/L.

### Exemple 4 - fluoration continue en phase liquide du 1230za avec un ratio molaire compris entre 9 et 10 à l'entrée du réacteur

La procédure de l'exemple 3 est reproduite, mais en doublant les débits d'alimentation d'organique et d' HF, soit 40g/h de 1230za et 40-44g/h de HF. Le ratio molaire d' HF sur le composé organique est compris entre 9 et 10.

La composition du flux de sortie est suivie par analyse GPC, et reportée dans le tableau 3 :

**Tableau 3 - composition molaire du gaz de sortie (débit d'entrée de 1230za de 40g/h)**

| Temps | Composition molaire en sortie | | | |
|---|---|---|---|---|
| | F1233zd-E | F1233zd-Z | F1234ze(E+Z) | F245fa |
| 5,5 h | 92,5 % | 3,8 % | 1,0 % | 1,0 % |
| 23,1 h | 93,7 % | 3,7 % | 0,6 % | 0,7 % |
| 29,1 h | 93,5 % | 3,8 % | 0,5 % | 0,6 % |
| 46,6 h | 91,4 % | 4,5 % | 0,2 % | 0,1 % |

La quantité d'impuretés surfluorées 1234ze (E+Z) et 245fa est rapidement inférieure à 1%.

La productivité du système réactionnel en F1233zd-E est de 0,68 mol/h/L.

A la suite des essais décrits dans les exemples 3 et 4, le réacteur a été vidangé. Les hydracides ont été piégés dans de l'eau, les organiques légers ont été piégés à froid et les organiques restants dans le pied de réacteur récupérés. Le niveau de liquide dans le réacteur a diminué au cours de l'essai et la composition du réacteur est la suivante: 11,3g de HF, 5,6g de HCl, 9 g d'organiques légers et 127g de composés organiques accumulés dans le réacteur. L'analyse chromatographique de ces deux fractions a été réalisée et a permis de reconstituer la composition globale du mélange liquide en pourcentage massique : 7,4% HF, 3,7%HCl, 3,5% E-1233zd, 0,25% Z-1233zd, 18,2% de 1230za, 2,4% de 1231, 14,8% de 1232, 49,2% de composés non identifiés.

La conversion de l'ensemble de l'essai est calculée sur la base de 27,9g de 1230za retrouvée dans la phase liquide par rapport à 3059 g introduit au total, soit 99,1%. La sélectivité en E-1233zd sur l'ensemble de l'essai continu (exemple 3 et exemple 4) est de 89,2%, la sélectivité en Z-1233zd est de 3,8%, 2% en 1232, 0,7% en 1234zeE, 0,7% en 245fa et 2,9% en produits inconnus.

### Exemple 5 - fluoration continue en phase liquide du 1230za avec un ratio molaire de 11

La procédure de l'exemple 3 est reproduite. Une quantité de 302g d'HF est introduite dans l'autoclave. La température du réacteur est ajustée à 90°C dans la phase liquide. La régulation de la pression est effectuée à 10 bars abs. Le rapport molaire d'HF sur le composé organique est ajusté à 11 et un essai de plus longue durée a été mis en œuvre pour établir la stabilité du procédé continu pendant 366h. 50 g/h de 1230za et 62 g/h d'HF sont introduits en continu. L'établissement d'un équilibre massique correct entre l'entrée et la sortie est régulièrement vérifié.

La composition du flux de sortie est suivie par analyse GPC, et reportée dans le tableau 4 :

**Tableau 4 - composition molaire du gaz de sortie (ratio molaire de 11, essai de plus longue durée)**

| Temps | Composition molaire en sortie | | | |
|---|---|---|---|---|
| | F1233zd-E | F1233zd-Z | F1234ze(E+Z) | F245fa |
| 3.5 h | 90,1% | 3,5% | 1,6% | 3,8% |
| 8.6 h | 93,3% | 3,5% | 0.9% | 0,9% |
| 14 h | 93,3% | 3,6% | 1,1% | 0,6% |
| 19 h | 93,3% | 3,6% | 0,6% | 0,6% |
| 24 h | 93,5% | 3,5% | 0,7% | 0,6% |
| 30 h | 93,5% | 3,6% | 0,7% | 0,6% |
| 36 h | 93,5% | 3,6% | 0.7% | 0,6% |
| 105 h | 93,5% | 3,6% | 0,6% | 0,5% |
| 111 h | 93,4% | 3,6% | 0.6% | 0,5% |
| 128 h | 93,5% | 3,6% | 0.5% | 0,5% |
| 135 h | 93,4% | 3,6% | 0,5% | 0,5% |
| 151 h | 93,7% | 3,6% | 0,4% | 0,4% |
| 158 h | 93,6% | 3,5% | 0,3% | 0,3% |
| 175 h | 93,1% | 3,6% | 0,3% | 0,2% |
| 250 h | 92,7% | 3,7% | 0,3% | 0,2% |
| 271 h | 92,7% | 3,7% | 0,3% | 0,2% |
| 278 h | 93,1% | 3,8% | 0,3% | 0,2% |
| 366 h | 91,9% | 3,9% | 0,2% | 0,04% |

Le complément de la composition est constitué de produits intermédiaires (1231, 1232, 242, 243) et / ou de produits non identifiés.

A la suite des essais décrits dans l'exemple 5, le réacteur a été vidangé. Les hydracides ont été piégés dans de l'eau, les organiques légers ont été piégés à froid et les organiques restants dans le pied de réacteur récupérés. Aucune trace de 1230za n'a pu être détectée. La conversion est donc de 100%. La sélectivité en E-1233zd est 90-92%. La productivité en E-1233zd est de 0,9 mol/h/l.

229g de substances organiques ont été collectées dans le fond du réacteur. Considérant que 17.6kgs de 1230za ont été alimentés pendant l'essai, la production de lourds est calculée à seulement 1,3%.

La quantité d'impuretés surfluorées 1234ze (E+Z) et 245fa est inférieure à 1%.

### Exemple 6 - distillation du E-1233zd brut

Un lot de 902g de produit brut collecté pendant une certaine période de l'essai continu de l'exemple 5 a été analysé avant d'être distillé. La colonne de distillation de type Oldershaw dispose de vingt plateaux. La distillation est effectuée à pression atmosphérique. Le produit est chargé à froid (5°C) dans le bouilleur qui est rechauffé progressivement de manière à soutirer différentes fractions. L'analyse du produit brut et des différentes fractions obtenues sont donnés dans le tableau 5.

**Tableau 5- Purification du E-1233zd brut par distillation**

| | Produit brut | Fraction 1 | Fraction 2 | Fraction 3 | Fraction 4 |
|---|---|---|---|---|---|
| E-1234ze | 0,279 | 1,249 | 0,001 | Nd | Nd |
| 245fa | 0,487 | 1,362 | 0,142 | 0,004 | Nd |
| Z-1234ze | 0,071 | 0,231 | 0,007 | Nd | Nd |
| E-1233zd | 93,85 | 97,047 | 99,831 | 99,967 | 99,898 |
| Z-1233zd | 3,458 | 0,053 | 0,019 | 0,025 | 0,102 |
| E-1232zd | 1,424 | 0,003 | Nd | 0,004 | Nd |
| 243fc | 0,062 | Nd | Nd | Nd | Nd |
| 1232za | 0,119 | Nd | Nd | Nd | Nd |
| X | 0,250 | 0,055 | Nd | Nd | Nd |

La pureté de la fraction 3 atteint une pureté supérieure à 99.9% avec un simple équipement de laboratoire. Le 245fa dans le produit final n'excède pas 40 ppm.

## Revendications

1. Procédé de fabrication du E-1-chloro-3,3,3-trifluoropropène comprenant au moins une étape au cours de laquelle le 1,1,3,3-tetrachloropropène réagit avec de l'acide fluorhydrique anhydre en phase liquide, en l'absence de catalyseur, avec un ratio molaire HF/1,1,3,3-tetrachloropropène compris entre 9 et 20, à une température comprise entre 50 et 150°C et une pression absolue comprise entre 1 et 20 bar, **caractérisé en ce que** la réaction de fluoration est mise en œuvre dans un réacteur non agité.

2. Procédé selon la revendication 1 **caractérisé en ce que** le ratio molaire HF/1,1,3,3-tetrachloropropène compris entre 9 et 15 et avantageusement entre 9 et 12.

3. Procédé selon l'une quelconque des revendications précédentes **caractérisé en ce que** la température est comprise entre 50 et 150°C, de préférence comprise entre 80 et 120°C et avantageusement entre 90 et 110°C.

4. Procédé selon l'une quelconque des revendications précédentes **caractérisé en ce que** la pression est entre 7 et 12 bar.

5. Procédé selon l'une quelconque des revendications précédentes **caractérisé en ce que** le procédé est mis en œuvre en continu, discontinu ou batch, de préférence en continu.

## Patentansprüche

1. Verfahren zur Herstellung von E-1-Chlor-3,3,3-trifluorpropen, das mindestens einen Schritt umfasst, bei dem 1,1,3,3-Tetrachlorpropen in der Flüssigphase in Abwesenheit von Katalysator mit wasserfreiem Chlorwasserstoff in einem HF/1,1,3,3-Tetrachlorpropen-Molverhältnis zwischen 9 und 20 bei einer Temperatur zwischen 50 und 150 °C und einem Absolutdruck zwischen 1 und 20 bar reagiert, **dadurch gekennzeichnet, dass** die Fluorierungsreaktion in einem nicht gerührten Reaktor durchgeführt wird.

2. Verfahren nach Anspruch 1, **dadurch gekennzeichnet, dass** das HF/1,1,3,3-Tetrachlorpropen-Molverhältnis zwischen 9 und 15 und vorteilhafterweise zwischen 9 und 12 liegt.

3. Verfahren nach einem der vorhergehenden Ansprüche, **dadurch gekennzeichnet, dass** die Temperatur zwischen 50 und 150 °C, vorzugsweise zwischen 80 und 120 °C und vorteilhafterweise zwischen 90 und 110 °C liegt.

4. Verfahren nach einem der vorhergehenden Ansprüche, **dadurch gekennzeichnet, dass** der Druck zwischen 7 und 12 bar liegt.

5. Verfahren nach einem der vorhergehenden Ansprüche, **dadurch gekennzeichnet, dass** das Verfahren kontinuierlich, diskontinuierlich oder chargenweise, vorzugsweise kontinuierlich, durchgeführt wird.

## Claims

1. Process for the manufacture of E-1-chloro-3,3,3-trifluoropropene comprising at least one stage during which 1,1,3,3-tetrachloropropene reacts with anhydrous hydrofluoric acid in the liquid phase, in the absence of catalyst, with an HF/1,1,3,3-tetrachloropropene molar ratio of between 9 and 20, at a temperature of between 50°C and 150°C and an absolute pressure of between 1 and 20 bar, **characterized in that** the fluorination reaction is carried out in an unstirred reactor.

2. Process according to Claim 1, **characterized in that** the HF/1,1,3,3-tetrachloropropene molar ratio is between 9 and 15 and advantageously between 9 and 12.

3. Process according to either one of the preceding claims, **characterized in that** the temperature is between 50°C and 150°C, preferably between 80°C and 120°C and advantageously between 90°C and 110°C.

4. Process according to any one of the preceding claims, **characterized in that** the pressure is between 7 and 12 bar.

5. Process according to any one of the preceding claims, **characterized in that** the process is carried out continuously, noncontinuously or batchwise, preferably continuously.
